# EUROPEAN PATENT SPECIFICATION

(11) **EP 3 155 987 B1**
(45) Date of publication and mention of the grant of the patent: **18.09.2019**
(21) Application number: 16193980.6
(22) Date of filing: 14.10.2016
(51) Int. Cl.: A61B 17/072

(54) **SURGICAL STAPLER WITH PROGRESSIVELY DRIVEN ASYMMETRIC ALTERNATING STAPLE DRIVERS**
CHIRURGISCHES KLAMMERGERÄT MIT PROGRESSIV ANGETRIEBENEM, ASYMMETRISCHEM, WECHSELNDEM KLAMMERTREIBER
AGRAFEUSE CHIRURGICALE AVEC GUIDES ALTERNATIFS D'AGRAFE À ENTRAINEMENT ALTERNATIF PROGRESSIF

(30) Priority: 15.10.2015 US 201514884073
(43) Date of publication of application: 19.04.2017
(73) Proprietor: Ethicon LLC, 00969 Guaynabo (PR)
(72) Inventor: SCHEIB, Charles J., Cincinnati, OH 45242 (US); SHELTON, IV, Frederick E., Cincinnati, OH 45242 (US)
(74) Representative: Carpmaels & Ransford LLP

(56) References cited:
- EP-A1- 2 886 070
- US-A1- 2014 239 044

## Description

### BACKGROUND

In some settings, endoscopic surgical instruments may be preferred over traditional open surgical devices since a smaller incision may reduce the post-operative recovery time and complications. Consequently, some endoscopic surgical instruments may be suitable for placement of a distal end effector at a desired surgical site through the cannula of a trocar. These distal end effectors may engage tissue in a number of ways to achieve a diagnostic or therapeutic effect (e.g., endocutter, grasper, cutter, stapler, clip applier, access device, drug/gene therapy delivery device, and energy delivery device using ultrasonic vibration, RF, laser, etc.). Endoscopic surgical instruments may include a shaft between the end effector and a handle portion, which is manipulated by the clinician. Such a shaft may enable insertion to a desired depth and rotation about the longitudinal axis of the shaft, thereby facilitating positioning of the end effector within the patient. Positioning of an end effector may be further facilitated through inclusion of one or more articulation joints or features, enabling the end effector to be selectively articulated or otherwise deflected relative to the longitudinal axis of the shaft.

Examples of endoscopic surgical instruments include surgical staplers. Some such staplers are operable to clamp down on layers of tissue, cut through the clamped layers of tissue, and drive staples through the layers of tissue to substantially seal the severed layers of tissue together near the severed ends of the tissue layers. Merely exemplary surgical staplers are disclosed in U.S. Pat. No. 4,805,823, entitled "Pocket Configuration for Internal Organ Staplers," issued February 21, 1989; U.S. Pat. No. 5,415,334, entitled "Surgical Stapler and Staple Cartridge," issued May 16, 1995; U.S. Pat. No. 5,465,895, entitled "Surgical Stapler Instrument," issued November 14, 1995; U.S. Pat. No. 5,597,107, entitled "Surgical Stapler Instrument," issued January 28, 1997; U.S. Pat. No. 5,632,432, entitled "Surgical Instrument," issued May 27, 1997; U.S. Pat. No. 5,673,840, entitled "Surgical Instrument," issued October 7, 1997; U.S. Pat. No. 5,704,534, entitled "Articulation Assembly for Surgical Instruments," issued January 6, 1998; U.S. Pat. No. 5,814,055, entitled "Surgical Clamping Mechanism," issued September 29, 1998; U.S. Pat. No. 6,978,921, entitled "Surgical Stapling Instrument Incorporating an E-Beam Firing Mechanism," issued December 27, 2005; U.S. Pat. No. 7,000,818, entitled "Surgical Stapling Instrument Having Separate Distinct Closing and Firing Systems," issued February 21, 2006; U.S. Pat. No. 7,143,923, entitled "Surgical Stapling Instrument Having a Firing Lockout for an Unclosed Anvil," issued December 5, 2006; U.S. Pat. No. 7,303,108, entitled "Surgical Stapling Instrument Incorporating a Multi-Stroke Firing Mechanism with a Flexible Rack," issued December 4, 2007; U.S. Pat. No. 7,367,485, entitled "Surgical Stapling Instrument Incorporating a Multistroke Firing Mechanism Having a Rotary Transmission," issued May 6, 2008; U.S. Pat. No. 7,380,695, entitled "Surgical Stapling Instrument Having a Single Lockout Mechanism for Prevention of Firing," issued June 3, 2008; U.S. Pat. No. 7,380,696, entitled "Articulating Surgical Stapling Instrument Incorporating a Two-Piece E-Beam Firing Mechanism," issued June 3, 2008; U.S. Pat. No. 7,404,508, entitled "Surgical Stapling and Cutting Device," issued July 29, 2008; U.S. Pat. No. 7,434,715, entitled "Surgical Stapling Instrument Having Multistroke Firing with Opening Lockout," issued October 14, 2008; U.S. Pat. No. 7,721,930, entitled "Disposable Cartridge with Adhesive for Use with a Stapling Device," issued May 25, 2010; U.S. Pat. No. 8,408,439, entitled "Surgical Stapling Instrument with An Articulatable End Effector," issued April 2, 2013; and U.S. Pat. No. 8,453,914, entitled "Motor-Driven Surgical Cutting Instrument with Electric Actuator Directional Control Assembly," issued June 4, 2013.

While the surgical staplers referred to above are described as being used in endoscopic procedures, it should be understood that such surgical staplers may also be used in open procedures and/or other non-endoscopic procedures. By way of example only, a surgical stapler may be inserted through a thoracotomy, and thereby between a patient's ribs, to reach one or more organs in a thoracic surgical procedure that does not use a trocar as a conduit for the stapler. Such procedures may include the use of the stapler to sever and close a vessel leading to a lung. For instance, the vessels leading to an organ may be severed and closed by a stapler before removal of the organ from the thoracic cavity. Of course, surgical staplers may be used in various other settings and procedures.

Examples of surgical staplers that may be particularly suited or use through a thoracotomy are disclosed in U.S. Patent App. No. 14/810,786, entitled "Surgical Staple Cartridge with Compression Feature at Knife Slot," filed July 29, 2015; U.S. Patent Pub. No. 2014/0243801, entitled "Surgical Instrument End Effector Articulation Drive with Pinion and Opposing Racks," published August 28, 2014; U.S. Patent Pub. No. 2014/0239041, entitled "Lockout Feature for Movable Cutting Member of Surgical Instrument," published August 28, 2014; U.S. Patent Pub. No. 2014/0239042, entitled "Integrated Tissue Positioning and Jaw Alignment Features for Surgical Stapler," published August 28, 2014; U.S. Patent Pub. No. 2014/0239036, entitled "Jaw Closure Feature for End Effector of Surgical Instrument," published August 28, 2014; U.S. Patent Pub. No. 2014/0239040, entitled "Surgical Instrument with Articulation Lock having a Detenting Binary Spring," published August 28, 2014; U.S. Patent Pub. No. 2014/0239043, entitled "Distal Tip Features for End Effector of Surgical Instrument," published August 28, 2014; U.S. Patent Pub. No. 2014/0239037, entitled "Staple Forming Features for Surgical Stapling Instrument," published August 28, 2014; U.S. Patent Pub. No. 2014/0239038, entitled "Surgical Instrument with Multi-Diameter Shaft," published August 28, 2014; and U.S. Patent Pub. No. 2014/0239044, entitled "Installation Features for Surgical Instrument End Effector Cartridge," published August 28, 2014.

While various kinds of surgical stapling instruments and associated components have been made and used, it is believed that no one prior to the inventor(s) has made or used the invention described in the appended claims.

### BRIEF DESCRIPTION OF THE DRAWINGS

The accompanying drawings, which are incorporated in and constitute a part of this specification, illustrate embodiments of the invention, and, together with the general description of the invention given above, and the detailed description of the embodiments given below, serve to explain the principles of the present invention.
FIG. 1 depicts a perspective view of an exemplary articulating surgical stapling instrument;
FIG. 2 depicts a side elevational view of the instrument of FIG. 1;
FIG. 3 depicts a perspective view of an end effector of the instrument of FIG. 1, with the end effector in a closed configuration;
FIG. 4 depicts a perspective view of the end effector of FIG. 3, with the end effector in an open configuration;
FIG. 5 depicts an exploded perspective view of the end effector of FIG. 3;
FIG. 6 depicts a cross-sectional end view of the end effector of FIG. 3, taken along line 6-6 of FIG. 4;
FIG. 7A depicts a cross-sectional side view of the end effector of FIG. 3, taken along line 7-7 of FIG. 4, with a firing beam in a proximal position;
FIG. 7B depicts a cross-sectional side view of the end effector of FIG. 3, taken along line 7-7 of FIG. 4, with the firing beam in a distal position;
FIG. 8 depicts a perspective view of the end effector of FIG. 3, positioned at tissue and having been actuated once in the tissue;
FIG. 9 depicts a side elevational view of another exemplary articulating surgical stapling instrument;
FIG. 10 depicts a perspective view of an end effector of the instrument of FIG. 9, with the end effector in an open configuration;
FIG. 11 depicts a top view of a lower jaw of the end effector of FIG. 10;
FIG. 12 depicts a bottom view of an upper jaw of the end effector of FIG. 10;
FIG. 13 depicts an exploded perspective view of the lower jaw of FIG. 10;
FIG. 14 depicts a perspective view of a wedge sled of the lower jaw shown in FIG. 13;
FIG. 15 depicts a right perspective view of a triple driver assembly of the lower jaw of FIG. 10;
FIG. 16 depicts a right perspective view of another triple driver assembly of the lower jaw of FIG. 10;
FIG. 17 depicts atop view of an arrangement of triple driver assemblies of the lower jaw of FIG. 10;
FIG. 18A depicts a side cross-sectional view of the wedge sled of FIG. 14 at a first longitudinal position, sliding toward the triple driver assemblies of FIG. 17, taken generally along a centerline of the lower jaw of FIG. 13;
FIG. 18B depicts a side cross-sectional view of the wedge sled of FIG. 14 at a second longitudinal position, with the triple driver assemblies of FIG. 17 in an upper position, taken generally along a centerline of the lower jaw of FIG. 13;
FIG. 19 depicts a perspective view of the lower jaw of FIG. 13, in partial cross-section taken along section line 19-19 of FIG. 11;
FIG. 20A depicts a schematic representation of a liver having a vessel extending through the liver tissue;
FIG. 20B depicts the schematic representation of the end effector of FIG. 10 severing the liver tissue of FIG. 20A;
FIG. 20C depicts the schematic representation of the vessel of FIG. 20B exposed from the severed liver tissue of FIG. 20A;
FIG. 20D depicts the schematic representation of the end effector of FIG. 10 stapling the exposed vessel of FIG. 20C; and
FIG. 20E depicts the schematic representation of the liver tissue of FIG. 20A having a portion of the liver tissue and the vessel resected therefrom.

The drawings are not intended to be limiting in any way, and it is contemplated that various embodiments of the invention may be carried out in a variety of other ways, including those not necessarily depicted in the drawings. The accompanying drawings incorporated in and forming a part of the specification illustrate several aspects of the present invention, and together with the description serve to explain the principles of the invention; it being understood, however, that this invention is not limited to the precise arrangements shown.

### DETAILED DESCRIPTION

The following description of certain examples of the invention should not be used to limit the scope of the present invention. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the invention. Accordingly, the drawings and descriptions should be regarded as illustrative in nature and not restrictive.

### I. Exemplary Surgical Stapler

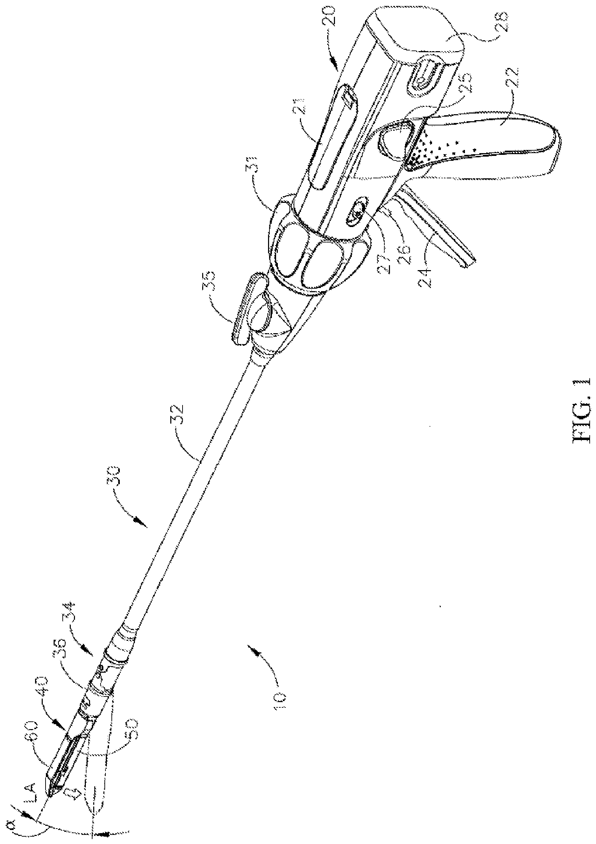

FIG. 1 depicts an exemplary surgical stapling and severing instrument (10) that includes a handle assembly (20), a shaft assembly (30), and an end effector (40). End effector (40) and the distal portion of shaft assembly (30) are sized for insertion, in a nonarticulated state as depicted in FIG. 1, through a trocar cannula to a surgical site in a patient for performing a surgical procedure. By way of example only, such a trocar may be inserted in a patient's abdomen, between two of the patient's ribs, or elsewhere. In some settings, instrument (10) is used without a trocar. For instance, end effector (40) and the distal portion of shaft assembly (30) may be inserted directly through a thoracotomy or other type of incision. It should be understood that terms such as "proximal" and "distal" are used herein with reference to a clinician gripping handle assembly (20) of instrument (10). Thus, end effector (40) is distal with respect to the more proximal handle assembly (20). It will be further appreciated that for convenience and clarity, spatial terms such as "vertical" and "horizontal" are used herein with respect to the drawings. However, surgical instruments are used in many orientations and positions, and these terms are not intended to be limiting and absolute.

### A. Exemplary Handle Assembly and Shaft Assembly

As shown in FIGS. 1-2, handle assembly (20) of the present example comprises pistol grip (22), a closure trigger (24), and a firing trigger (26). Each trigger (24, 26) is selectively pivotable toward and away from pistol grip (22) as will be described in greater detail below. Handle assembly (20) further includes an anvil release button (25), a firing beam reverse switch (27), and a removable battery pack (28). These components will also be described in greater detail below. Of course, handle assembly (20) may have a variety of other components, features, and operabilities, in addition to or in lieu of any of those noted above. Other suitable configurations for handle assembly (20) will be apparent to those of ordinary skill in the art in view of the teachings herein.

As shown in FIGS. 1-3, shaft assembly (30) of the present example comprises an outer closure tube (32), an articulation section (34), and a closure ring (36), which is further coupled with end effector (40). Closure tube (32) extends along the length of shaft assembly (30). Closure ring (36) is positioned distal to articulation section (34). Closure tube (32) and closure ring (36) are configured to translate longitudinally relative to handle assembly (20). Longitudinal translation of closure tube (32) is communicated to closure ring (36) via articulation section (34). Exemplary features that may be used to provide longitudinal translation of closure tube (32) and closure ring (36) will be described in greater detail below.

Articulation section (34) is operable to laterally deflect closure ring (36) and end effector (40) laterally away from the longitudinal axis (LA) of shaft assembly (30) at a desired angle (α). End effector (40) may thereby reach behind an organ or approach tissue from a desired angle or for other reasons. In some versions, articulation section (34) enables deflection of end effector (40) along a single plane. In some other versions, articulation section (34) enables deflection of end effector along more than one plane. In the present example, articulation is controlled through an articulation control knob (35) which is located at the proximal end of shaft assembly (30). Knob (35) is rotatable about an axis that is perpendicular to the longitudinal axis (LA) of shaft assembly (30). Closure ring (36) and end effector (40) pivot about an axis that is perpendicular to the longitudinal axis (LA) of shaft assembly (30) in response to rotation of knob (35). By way of example only, rotation of knob (35) clockwise may cause corresponding clockwise pivoting of closure ring (36) and end effector (40) at articulation section (34). Articulation section (34) is configured to communicate longitudinal translation of closure tube (32) to closure ring (36), regardless of whether articulation section (34) is in a straight configuration or an articulated configuration.

In some versions, articulation section (34) and/or articulation control knob (35) are/is constructed and operable in accordance with at least some of the teachings of U.S. Pub. No. 2014/0243801, entitled "Surgical Instrument End Effector Articulation Drive with Pinion and Opposing Racks," published August 28, 2014. Articulation section (34) may also be constructed and operable in accordance with at least some of the teachings of U.S. Pat. App. No. 14/314,125, entitled "Articulation Drive Features for Surgical Stapler," filed June 25, 2014; and/or in accordance with the various teachings below. Other suitable forms that articulation section (34) and articulation knob (35) may take will be apparent to those of ordinary skill in the art in view of the teachings herein.

As shown in FIGS. 1-2, shaft assembly (30) of the present example further includes a rotation knob (31). Rotation knob (31) is operable to rotate the entire shaft assembly (30) and end effector (40) relative to handle assembly (20) about the longitudinal axis (LA) of shaft assembly (30). In some versions, rotation knob (31) is operable to selectively lock the angular position of shaft assembly (30) and end effector (40) relative to handle assembly (20) about the longitudinal axis (LA) of shaft assembly (30). For instance, rotation knob (31) may be translatable between a first longitudinal position, in which shaft assembly (30) and end effector (40) are rotatable relative to handle assembly (20) about the longitudinal axis (LA) of shaft assembly (30); and a second longitudinal position, in which shaft assembly (30) and end effector (40) are not rotatable relative to handle assembly (20) about the longitudinal axis (LA) of shaft assembly (30). Of course, shaft assembly (30) may have a variety of other components, features, and operabilities, in addition to or in lieu of any of those noted above. By way of example only, at least part of shaft assembly (30) is constructed in accordance with at least some of the teachings of U.S. Pub. No. 2014/0239038, entitled "Surgical Instrument with Multi-Diameter Shaft," published August 28, 2014. Other suitable configurations for shaft assembly (30) will be apparent to those of ordinary skill in the art in view of the teachings herein.

### B. Exemplary End Effector

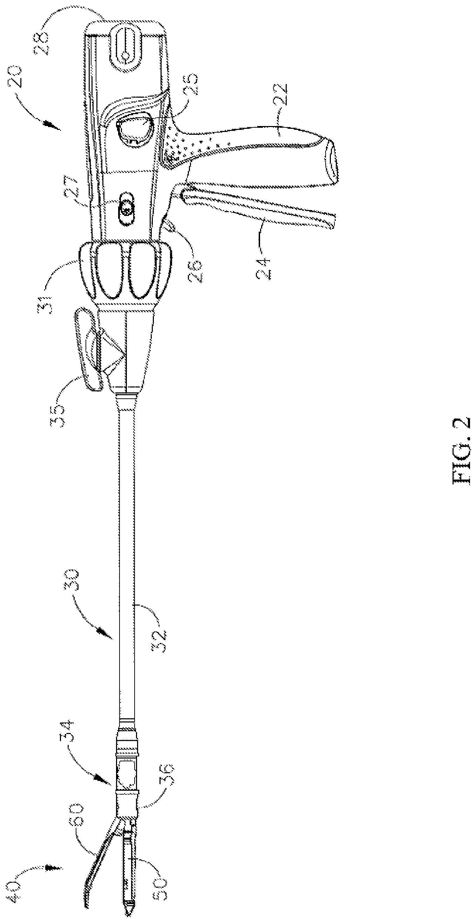

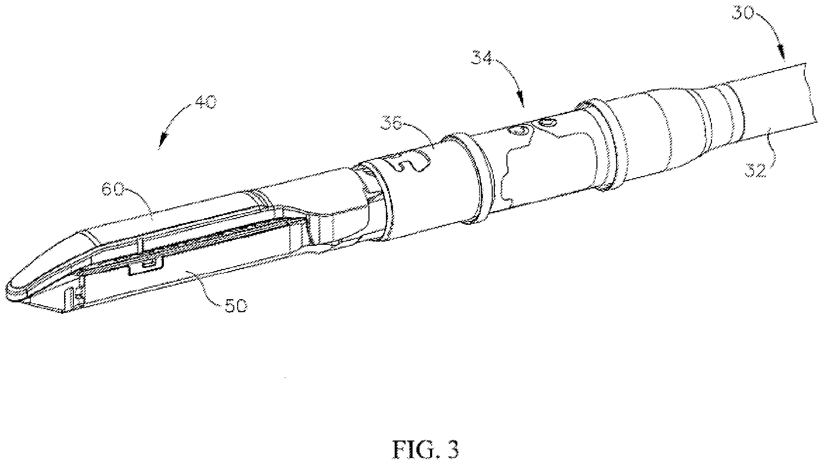

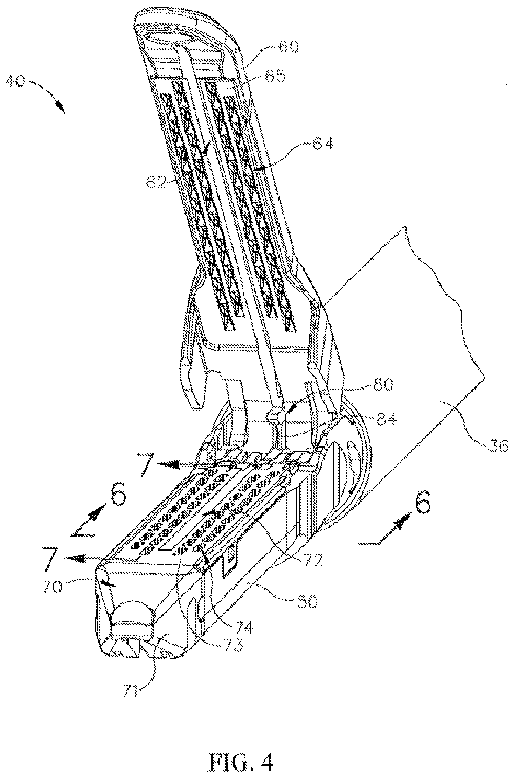

As also shown in FIGS. 3-5, end effector (40) of the present example includes a lower jaw (50) and a pivotable anvil (60). Anvil (60) includes a pair of integral, outwardly extending pins (66) that are disposed in corresponding curved slots (54) of lower jaw (50). Anvil (60) is pivotable toward and away from lower jaw (50) between an open position (shown in FIGS. 2 and 4) and a closed position (shown in FIGS. 1, 3, and 7A-7B). Use of the term "pivotable" (and similar terms with "pivot" as a base) should not be read as necessarily requiring pivotal movement about a fixed axis. For instance, in the present example, anvil (60) pivots about an axis that is defined by pins (66), which slide along curved slots (54) of lower jaw (50) as anvil (60) moves toward lower jaw (50). In such versions, the pivot axis translates along the path defined by slots (54) while anvil (60) simultaneously pivots about that axis. In addition or in the alternative, the pivot axis may slide along slots (54) first, with anvil (60) then pivoting about the pivot axis after the pivot axis has slid a certain distance along the slots (54). It should be understood that such sliding/translating pivotal movement is encompassed within terms such as "pivot," "pivots," "pivotal," "pivotable," "pivoting," and the like. Of course, some versions may provide pivotal movement of anvil (60) about an axis that remains fixed and does not translate within a slot or channel, etc.

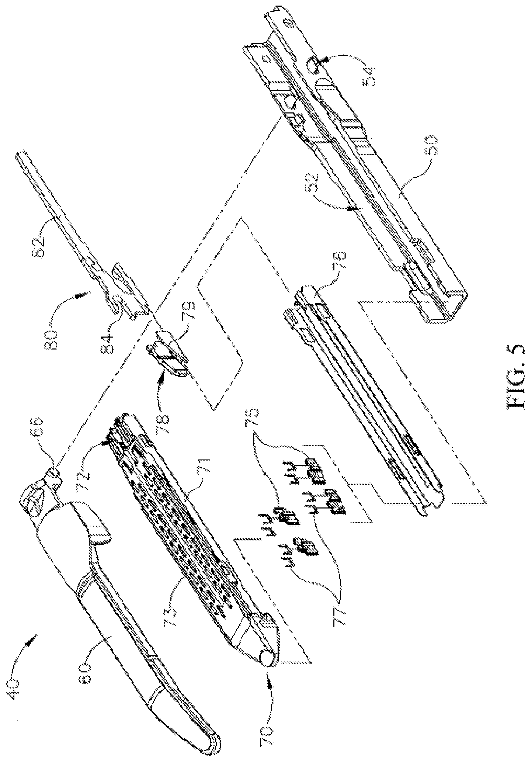

As best seen in FIG. 5, lower jaw (50) of the present example defines a channel (52) that is configured to receive a staple cartridge (70). Staple cartridge (70) may be inserted into channel (52), end effector (40) may be actuated, and then staple cartridge (70) may be removed and replaced with another staple cartridge (70). Lower jaw (50) thus releasably retains staple cartridge (70) in alignment with anvil (60) for actuation of end effector (40). In some versions, lower jaw (50) is constructed in accordance with at least some of the teachings of U.S. Pub. No. 2014/0239044, entitled "Installation Features for Surgical Instrument End Effector Cartridge," published August 28, 2014. Other suitable forms that lower jaw (50) may take will be apparent to those of ordinary skill in the art in view of the teachings herein.

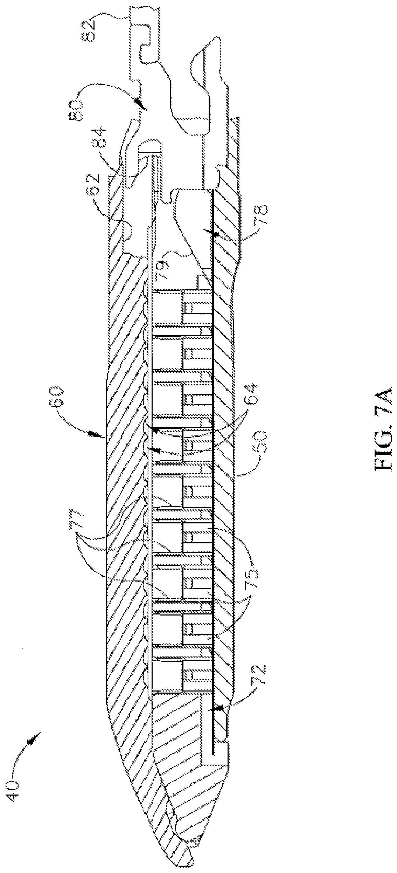

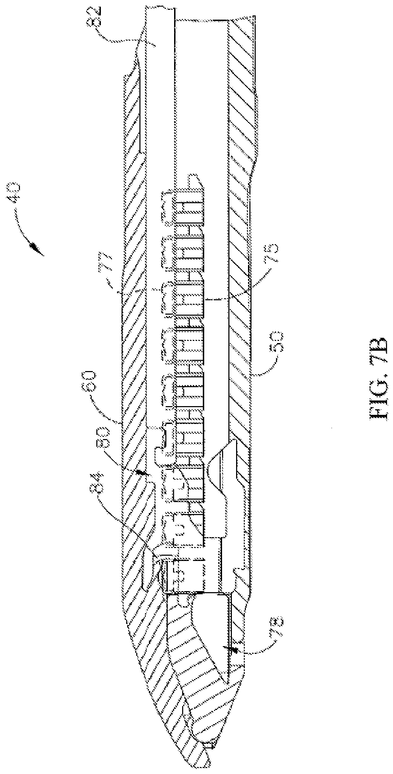

As best seen in FIGS. 4-6, staple cartridge (70) of the present example comprises a cartridge body (71) and a tray (76) secured to the underside of cartridge body (71). The upper side of cartridge body (71) presents a deck (73), against which tissue may be compressed when anvil (60) is in a closed position. Cartridge body (71) further defines a longitudinally extending channel (72) and a plurality of staple pockets (74). A staple (77) is positioned in each staple pocket (74). A staple driver (75) is also positioned in each staple pocket (74), underneath a corresponding staple (77), and above tray (76). As will be described in greater detail below, staple drivers (75) are operable to translate upwardly in staple pockets (74) to thereby drive staples (77) upwardly through staple pockets (74) and into engagement with anvil (60). Staple drivers (75) are driven upwardly by a wedge sled (78), which is captured between cartridge body (71) and tray (76), and which translates longitudinally through cartridge body (71). Wedge sled (78) includes a pair of obliquely angled cam surfaces (79), which are configured to engage staple drivers (75) and thereby drive staple drivers (75) upwardly as wedge sled (78) translates longitudinally through cartridge (70). For instance, when wedge sled (78) is in a proximal position as shown in FIG. 7A, staple drivers (75) are in downward positions and staples (77) are located in staple pockets (74). As wedge sled (78) is driven to the distal position shown in FIG. 7B by a translating knife member (80), wedge sled (78) drives staple drivers (75) upwardly, thereby driving staples (77) out of staple pockets (74) and into staple forming pockets (64). Thus, staple drivers (75) translate along a vertical dimension as wedge sled (78) translates along a horizontal dimension.

It should be understood that the configuration of staple cartridge (70) may be varied in numerous ways. For instance, staple cartridge (70) of the present example includes two longitudinally extending rows of staple pockets (74) on one side of channel (72); and another set of two longitudinally extending rows of staple pockets (74) on the other side of channel (72). However, in some other versions, staple cartridge (70) includes three, one, or some other number of staple pockets (74) on each side of channel (72). In some versions, staple cartridge (70) is constructed and operable in accordance with at least some of the teachings of U.S. Pub. No. 2014/0239042, entitled "Integrated Tissue Positioning and Jaw Alignment Features for Surgical Stapler," published August 28, 2014. In addition or in the alternative, staple cartridge (70) may be constructed and operable in accordance with at least some of the teachings of U.S. Pub. No. 2014/0239044, entitled "Installation Features for Surgical Instrument End Effector Cartridge," published August 28, 2014. Other suitable forms that staple cartridge (70) may take will be apparent to those of ordinary skill in the art in view of the teachings herein.

As best seen in FIG. 4, anvil (60) of the present example comprises a longitudinally extending channel (62) and a plurality of staple forming pockets (64). Channel (62) is configured to align with channel (72) of staple cartridge (70) when anvil (60) is in a closed position. Each staple forming pocket (64) is positioned to lie over a corresponding staple pocket (74) of staple cartridge (70) when anvil (60) is in a closed position. Staple forming pockets (64) are configured to deform the legs of staples (77) when staples (77) are driven through tissue and into anvil (60). In particular, staple forming pockets (64) are configured to bend the legs of staples (77) to secure the formed staples (77) in the tissue. Anvil (60) may be constructed in accordance with at least some of the teachings of U.S. Pub. No. 2014/0239042, entitled "Integrated Tissue Positioning and Jaw Alignment Features for Surgical Stapler," published August 28, 2014; at least some of the teachings of U.S. Pub. No. 2014/0239036, entitled "Jaw Closure Feature for End Effector of Surgical Instrument," published August 28, 2014; and/or at least some of the teachings of U.S. Pub. No. 2014/0239037, entitled "Staple Forming Features for Surgical Stapling Instrument," published August 28, 2014. Other suitable forms that anvil (60) may take will be apparent to those of ordinary skill in the art in view of the teachings herein.

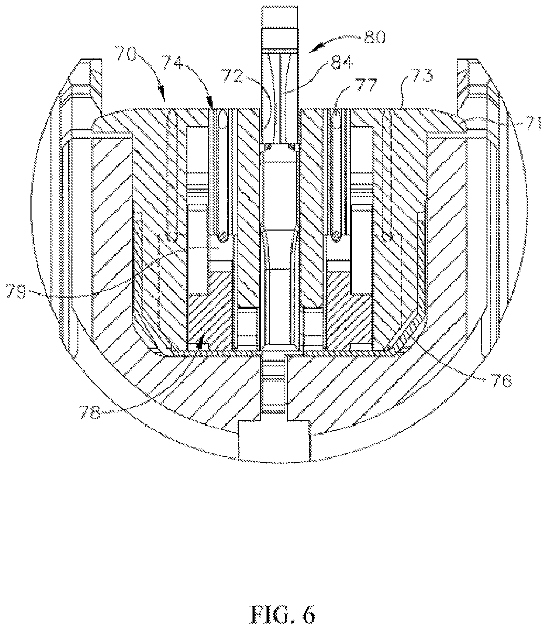

In the present example, knife member (80) is configured to translate through end effector (40). As best seen in FIGS. 5 and 7A-7B, knife member (80) is secured to the distal end of a firing beam (82), which extends through a portion of shaft assembly (30). As best seen in FIGS. 4 and 6, knife member (80) is positioned in channels (62, 72) of anvil (60) and staple cartridge (70). Knife member (80) includes a distally presented cutting edge (84) that is configured to sever tissue that is compressed between anvil (60) and deck (73) of staple cartridge (70) as knife member (80) translates distally through end effector (40). As noted above and as shown in FIGS. 7A-7B, knife member (80) also drives wedge sled (78) distally as knife member (80) translates distally through end effector (40), thereby driving staples (77) through tissue and against anvil (60) into formation. Various features that may be used to drive knife member (80) distally through end effector (40) will be described in greater detail below.

In some versions, end effector (40) includes lockout features that are configured to prevent knife member (80) from advancing distally through end effector (40) when a staple cartridge (70) is not inserted in lower jaw (50). In addition or in the alternative, end effector (40) may include lockout features that are configured to prevent knife member (80) from advancing distally through end effector (40) when a staple cartridge (70) that has already been actuated once (e.g., with all staples (77) deployed therefrom) is inserted in lower jaw (50). By way of example only, such lockout features may be configured in accordance with at least some of the teachings of U.S. Pub. No. 2014/0239041, entitled "Lockout Feature for Movable Cutting Member of Surgical Instrument," published August 28, 2014; and/or at least some of the teachings of U.S. Pat. App. No. 14/314,108, entitled "Method of Using Lockout Features for Surgical Staple cartridge," filed on June 25, 2014. Other suitable forms that lockout features may take will be apparent to those of ordinary skill in the art in view of the teachings herein. Alternatively, end effector (40) may simply omit such lockout features.

### C. Exemplary Actuation of Anvil

In the present example, anvil (60) is driven toward lower jaw (50) by advancing closure ring (36) distally relative to end effector (40). Closure ring (36) cooperates with anvil (60) through a camming action to drive anvil (60) toward lower jaw (50) in response to distal translation of closure ring (36) relative to end effector (40). Similarly, closure ring (36) may cooperate with anvil (60) to open anvil (60) away from lower jaw (50) in response to proximal translation of closure ring (36) relative to end effector (40). By way of example only, closure ring (36) and anvil (60) may interact in accordance with at least some of the teachings of U.S. Pub. No. 2014/0239036, entitled "Jaw Closure Feature for End Effector of Surgical Instrument," published August 28, 2014; and/or in accordance with at least some of the teachings of U.S. Patent App. No. 14/314,108, entitled "Jaw Opening Feature for Surgical Stapler," filed on June 25, 2014. Exemplary features that may be used to provide longitudinal translation of closure ring (36) relative to end effector (40) will be described in greater detail below.

As noted above, handle assembly (20) includes pistol grip (22) and closure trigger (24). As also noted above, anvil (60) is closed toward lower jaw (50) in response to distal advancement of closure ring (36). In the present example, closure trigger (24) is pivotable toward pistol grip (22) to drive closure tube (32) and closure ring (36) distally. Various suitable components that may be used to convert pivotal movement of closure trigger (24) toward pistol grip (22) into distal translation of closure tube (32) and closure ring (36) relative to handle assembly (20) will be apparent to those of ordinary skill in the art in view of the teachings herein. When closure trigger (24) reaches a fully pivoted state, such that anvil (60) is in a fully closed position relative to lower jaw (50), locking features in handle assembly (20) lock the position of closure trigger (24) and closure tube (32), thereby locking anvil (60) in a fully closed position relative to lower jaw (50). These locking features are released by actuation of anvil release button (25). Anvil release button (25) is configured and positioned to be actuated by the thumb of the operator hand that grasps pistol grip (22). In other words, the operator may grasp pistol grip (22) with one hand, actuate closure trigger (24) with one or more fingers of the same hand, and then actuate anvil release button (25) with the thumb of the same hand, without ever needing to release the grasp of pistol grip (22) with the same hand. Other suitable features that may be used to actuate anvil (60) will be apparent to those of ordinary skill in the art in view of the teachings herein.

### D. Exemplary Actuation of Firing Beam

In the present example, instrument (10) provides motorized control of firing beam (82). In particular, instrument (10) includes motorized components that are configured to drive firing beam (82) distally in response to pivoting of firing trigger (26) toward pistol grip (22). In some versions, a motor (not shown) is contained in pistol grip (22) and receives power from battery pack (28). This motor is coupled with a transmission assembly (not shown) that converts rotary motion of a drive shaft of the motor into linear translation of firing beam (82). In some such versions, firing beam (82) may only be advanced distally when anvil (60) is in a fully closed position relative to lower jaw (50). After firing beam (82) is advanced distally to sever tissue and drive staples (77) as described above with reference to FIGS. 7A-7B, the drive assembly for firing beam (82) may be automatically reversed to drive firing beam (82) proximally back to the retracted position (e.g., back from the position shown in FIG. 7B to the position shown in FIG. 7A). Alternatively, the operator may actuate firing beam reverse switch (27), which may reverse the drive assembly for firing beam (82) in order to retract firing beam (82) to a proximal position. Handle assembly (20) of the present example further includes a bailout feature (21), which is operable to provide a mechanical bailout allowing the operator to manually retract firing beam (82) proximally (e.g., in the event of power loss while firing beam (82) is in a distal position, etc.).

By way of example only, the features that are operable to provide motorized actuation of firing beam (82) may be configured and operable in accordance with at least some of the teachings of U.S. Pat. No. 8,210,411, entitled "Motor-Driven Surgical Instrument," issued July 3, 2012. As another merely illustrative example, the features that are operable to provide motorized actuation of firing beam (82) may be configured and operable in accordance with at least some of the teachings of U.S. Pat. No. 8,453,914, entitled "Motor-Driven Surgical Cutting Instrument with Electric Actuator Directional Control Assembly," issued June 4, 2013. As yet another merely illustrative example, the features that are operable to provide motorized actuation of firing beam (82) may be configured and operable in accordance with at least some of the teachings of U.S. Patent App. No. 14/226,142, entitled "Surgical Instrument Comprising a Sensor System," filed March 26, 2014.

Other suitable components, features, and configurations that may be used to provide motorization of firing beam (82) will be apparent to those of ordinary skill in the art in view of the teachings herein. It should also be understood that some other versions may provide manual driving of firing beam (82), such that a motor may be omitted. By way of example only, firing beam (82) may be manually actuated in accordance with at least some of the teachings of any other reference cited herein.

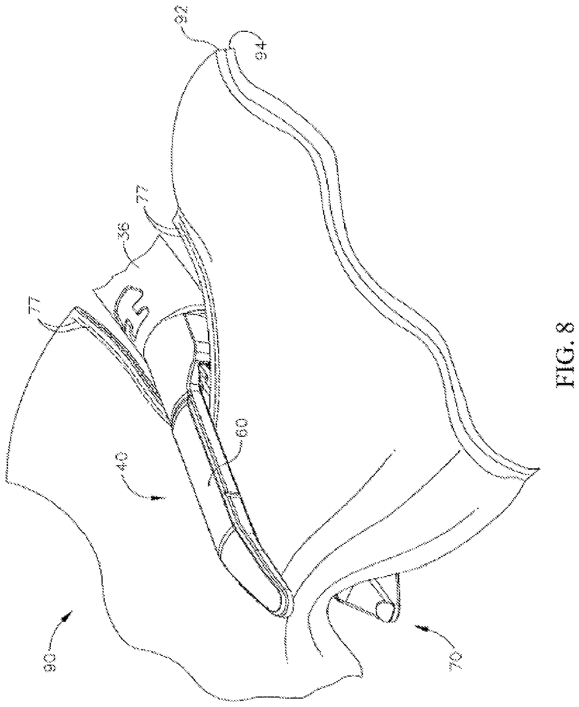

FIG. 8 shows end effector (40) having been actuated through a single stroke through tissue (90). As shown, cutting edge (84) (obscured in FIG. 8) has cut through tissue (90), while staple drivers (75) have driven two alternating rows of staples (77) through the tissue (90) on each side of the cut line produced by cutting edge (84). Staples (77) are all oriented substantially parallel to the cut line in this example, though it should be understood that staples (77) may be positioned at any suitable orientations. In the present example, end effector (40) is withdrawn from the trocar after the first stroke is complete, the spent staple cartridge (70) is replaced with a new staple cartridge (70), and end effector (40) is then again inserted through the trocar to reach the stapling site for further cutting and stapling. This process may be repeated until the desired amount of cuts and staples (77) have been provided. Anvil (60) may need to be closed to facilitate insertion and withdrawal through the trocar; and anvil (60) may need to be opened to facilitate replacement of staple cartridge (70).

It should be understood that cutting edge (84) may cut tissue substantially contemporaneously with staples (77) being driven through tissue during each actuation stroke. In the present example, cutting edge (84) just slightly lags behind driving of staples (77), such that staple (77) is driven through the tissue just before cutting edge (84) passes through the same region of tissue, though it should be understood that this order may be reversed or that cutting edge (84) may be directly synchronized with adjacent staples. While FIG. 8 shows end effector (40) being actuated in two layers (92, 94) of tissue (90), it should be understood that end effector (40) may be actuated through a single layer of tissue (90) or more than two layers (92, 94) of tissue. It should also be understood that the formation and positioning of staples (77) adjacent to the cut line produced by cutting edge (84) may substantially seal the tissue at the cut line, thereby reducing or preventing bleeding and/or leaking of other bodily fluids at the cut line. Furthermore, while FIG. 8 shows end effector (40) being actuated in two substantially flat, apposed planar layers (92, 94) of tissue, it should be understood that end effector (40) may also be actuated across a tubular structure such as a blood vessel, a section of the gastrointestinal tract, etc. FIG. 8 should therefore not be viewed as demonstrating any limitation on the contemplated uses for end effector (40). Various suitable settings and procedures in which instrument (10) may be used will be apparent to those of ordinary skill in the art in view of the teachings herein.

It should also be understood that any other components or features of instrument (10) may be configured and operable in accordance with any of the various references cited herein. Additional exemplary modifications that may be provided for instrument (10) will be described in greater detail below. Various suitable ways in which the below teachings may be incorporated into instrument (10) will be apparent to those of ordinary skill in the art. Similarly, various suitable ways in which the below teachings may be combined with various teachings of the references cited herein will be apparent to those of ordinary skill in the art. It should also be understood that the below teachings are not limited to instrument (10) or devices taught in the references cited herein. The below teachings may be readily applied to various other kinds of instruments, including instruments that would not be classified as surgical staplers. Various other suitable devices and settings in which the below teachings may be applied will be apparent to those of ordinary skill in the art in view of the teachings herein.

### II. Exemplary Alternative Stapling End Effector

While the above surgical instrument (10) provides one example of an end effector (40) that may be used to staple and sever tissue within a patient, it will be appreciated that the human body is comprised a wide variety of tissues located in distinct, sometimes difficult to access regions throughout the patient. For example, a liver includes tissue including vessels or ducts passing throughout. In settings where the liver includes a tumor, it may be desirable to resect the portion of the liver containing the tumor. The resection may be anatomic (e.g., resection of the right or left side of the liver, inclusive of the lobes on that side) or non-anatomic (e.g., resection of just a single lobe or wedge of liver tissue). This resection process may entail at least three kinds of steps - a first step to dissect the tissue (e.g., liver parenchyma) around the vessels or ducts, to thereby isolate or reveal the vessels or ducts; a second step to ligate those vessels or ducts; and a third step to sever the ligated vessels or ducts.

One such method of liver resection includes the well known Kelly clamp method, where a Kelly style clamp is used to compress the liver tissue and thereby dissect the tissue through a crushing action. However, treatments may require many instruments to accommodate such a wide variety of tissues and vessels or ducts within the human body, thereby adding to the time and complexity associated with assessing the state of the tissue, selecting and/or changing instruments, and performing the resection. It may therefore be desirable to provide a surgical instrument (410) with an end effector (412) having a pair of crush surfaces (414, 416) that are configured to sever tissue by crushing the tissue; while also providing an adjacent staple cartridge (418) to selectively ligate one or more vessels or ducts passing through the tissue. Thereby, a single surgical instrument (210, 410) will allow the operator to more quickly assess the tissue and proceed with further tissue dissection and/or ligation of vessels and ducts.

Surgical instruments (410) are described below in the context of dissecting liver tissue (e.g., liver parenchyma) with crush surfaces (414, 416) and using staples to ligate associated vessels or ducts (e.g., portal vein, hepatic vein branches, hepatic artery branches, extrahepatic vessels, etc.). In some instances (e.g., in the case of hepatic vein branches and hepatic artery branches, etc.), the vessel or duct that is sealed by the staples is exposed when the operator crushes the liver tissue with surfaces (414, 416). In some other instances (e.g., in the case of the portal vein and extrahepatic vessels, etc.), the vessel or duct that is sealed by the staples is separate from the liver tissue that the operator has crushed with surfaces (414, 416). While the following description of surgical instruments (410) and method of treatment is provided in the context of liver resection, it will be appreciated that surgical instruments (410) may be alternatively configured to treat any tissue in the human body with similar features. It should also be understood that that the features discussed below may be readily incorporated into surgical instrument (10) discussed above. To this end, like numbers indicate like features described above in greater detail.

In the following examples, end effectors (412) apply at least two laterally spaced apart rows of staples where the staples in one row have the same height as the staples in another row. In some variations, end effectors (412) are modified to apply at least two laterally spaced apart rows of staples where the staples in one row have a height that is different from the height of the staples in another row.

### A. Exemplary Stapling Instrument with Shortened Straight End Effector

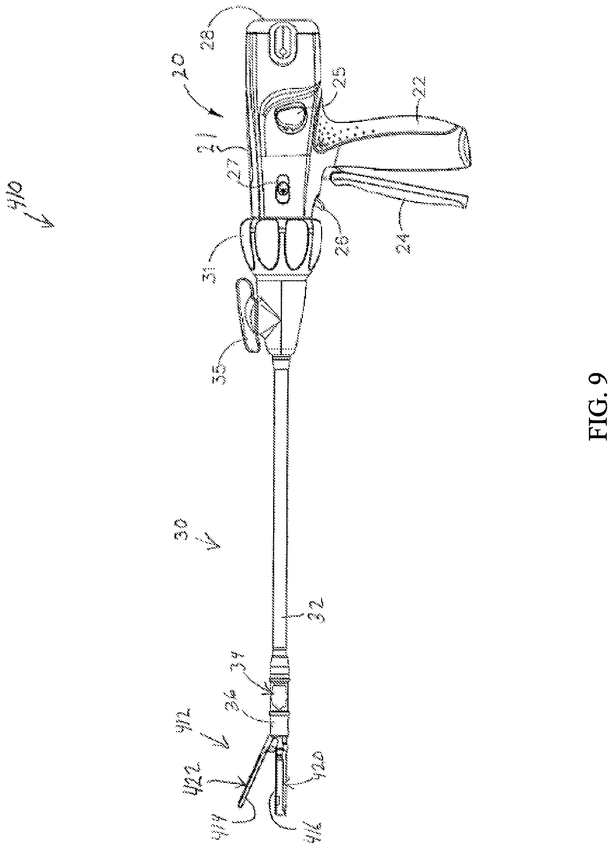

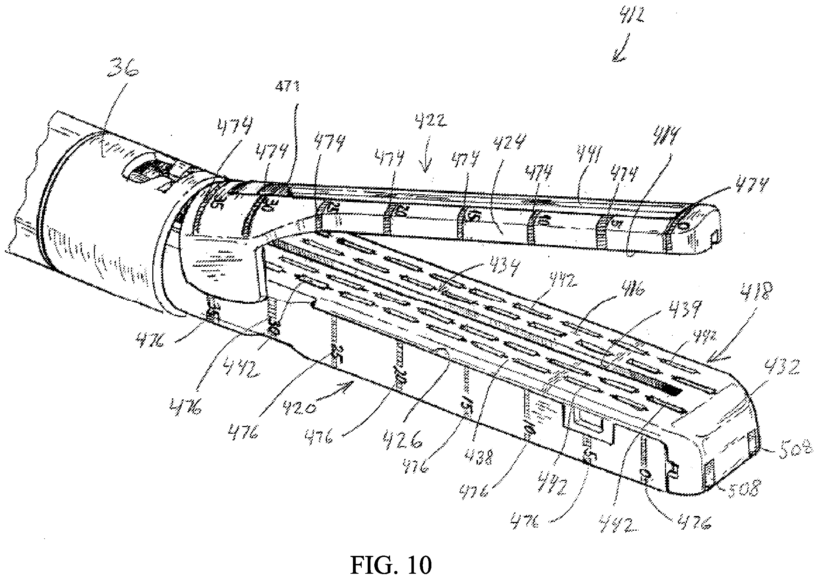

FIGS. 9-13 show surgical instrument (410) with end effector (412) having upper crush surface (414), lower crush surface (416), staple cartridge (418), and knife member (419). As noted above, it may be desirable to provide such a surgical instrument (410) with an end effector (412) having crush surfaces (414, 416) that are configured to sever tissue by crushing the tissue; while also providing adjacent staple cartridge (418) to selectively ligate one or more vessels passing through the tissue. In addition, knife member (419) is configured to cut the one or more vessels for complete removal of the surrounding tissue. Thereby, surgical instrument (410) will allow the operator to more quickly assess the tissue and proceed with further tissue severing and/or tissue ligation. Surgical instrument (410) of the present example also includes handle assembly (20) and shaft assembly (30) discussed above in greater detail. Except as otherwise described below, end effector (412), in conjunction with handle assembly (20) and shaft assembly (30), is configured and operable similar to end effector (40) *(see* FIG. 1). By way of example only, end effector (412) may have a length of approximately 40 mm and a width of approximately 7mm. Alternatively, any other suitable dimension may be used.

End effector (412) of the present example includes a lower jaw (420) and an upper jaw (422), which forms an anvil (424). Upper jaw (422) is pivotally mounted relative to lower jaw (420) for receiving the tissue therebetween. More particularly, anvil (424) is pivotable toward and away from lower jaw (420) between an open position and a closed position (e.g., in response to pivotal movement of trigger (24) toward and away from pistol grip (22)). For instance, in the present example, anvil (424) pivots about an axis that is defined by pins (not shown), which slide along curved slots (not shown) of lower jaw (420) as anvil (424) moves toward lower jaw (420). In such versions, the pivot axis translates along the path defined by slots (not shown) while anvil (424) simultaneously pivots about that axis. In addition or in the alternative, the pivot axis may slide along slots (not shown) first, with anvil (424) then pivoting about the pivot axis after the pivot axis slides a certain distance along the slots (not shown). Alternatively, some versions may provide pivotal movement of anvil (424) about an axis that remains fixed and does not translate within a slot or channel, etc.

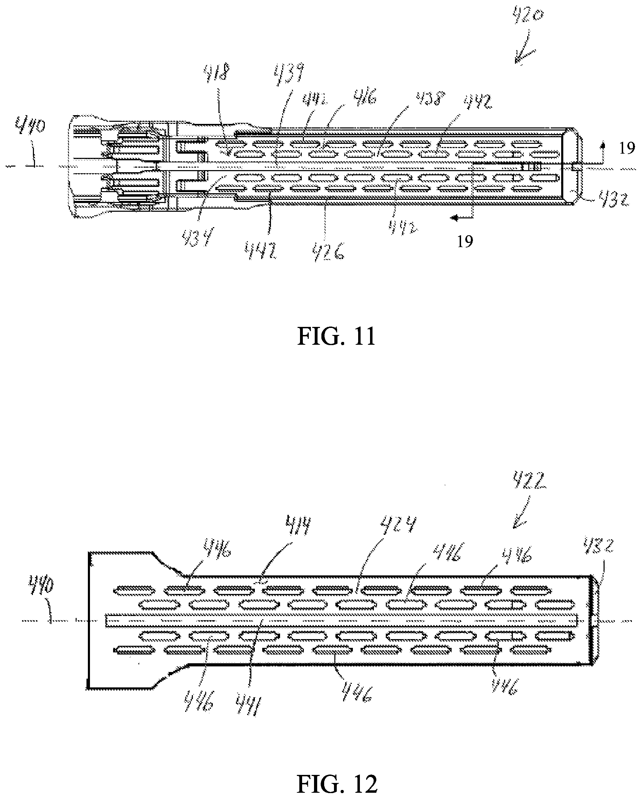

As best seen in FIGS. 11-13, lower jaw (420) of the present example defines a channel (426) that is configured to receive staple cartridge (418). Staple cartridge (418) may be inserted into channel (426), end effector (412) may be actuated, and then staple cartridge (418) may be removed and replaced with another staple cartridge (418). Lower jaw (420) thus releasably retains staple cartridge (418) in alignment with anvil (424) for actuation of end effector (412). In some alternative versions, the components of staple cartridge (418) are fully integrated into lower jaw (420) such that end effector (412) may only be used once. Other suitable forms that lower jaw (420) may take will be apparent to those of ordinary skill in the art in view of the teachings herein. In the present example, lower and upper jaws (420, 422) extend to a distal tip (432), which is further defined by staple cartridge (418).

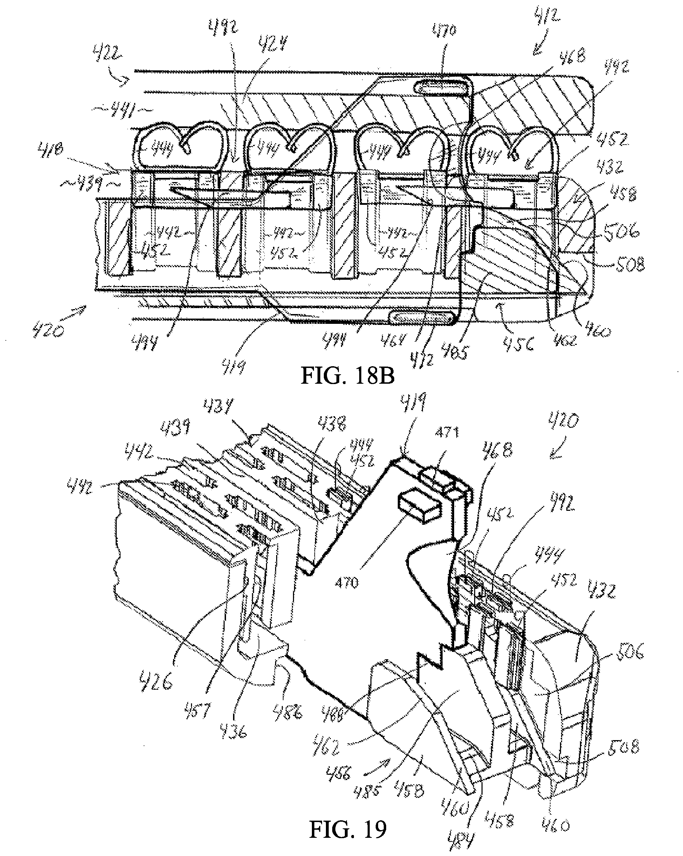

Staple cartridge (418) of the present example comprises a cartridge body (434) and a tray (436) (*see* FIG. 19) secured to an underside of cartridge body (434). An upper side of cartridge body (434) presents a deck (438), against which tissue may be compressed when anvil (424) is in a closed position. In the present example, lower crush surface (416) is positioned along staple cartridge (418). However, it will be appreciated that lower crush surface (416), as well as cooperating upper crush surface (414) may be alternatively positioned along end effector (412) for severing tissue via compression.

Cartridge body (434) further defines an elongated channel (439) extending through lower jaw (420) and linearly along a centerline (440) of end effector (412). Another elongated channel (441) defined by anvil (424) extends through upper jaw (422) and linearly along centerline (440), as well, for reasons discussed below in greater detail. A plurality of staple pockets (442) follow a predetermined pattern along deck (438) on opposing sides of centerline (440). More particularly, staple cartridge (418) includes two longitudinally extending rows of staple pockets (442) on one side of centerline (440); and another set of two longitudinally extending rows of staple pockets (442) on the other side of centerline (440). However, in some other versions, staple cartridge (418) may include three, one, or some other number of staple pockets (442) on each side of centerline (440).

One of a plurality of staples (444) is positioned in respective staple pockets (442). Adjacent rows of staple pockets (442) are configured to overlap in a direction transverse to the centerline (440) in order to install the plurality of staples (444) within the tissue and inhibit openings therebetween for improved ligation. In other words, a consistent gap (G1) is maintained between adjacent staple pockets (442) for consistent overlap in the present example. As used herein, the term "overlap" is intended to include one feature overlapping with another in at least one direction. Thus, a feature may be offset from another feature and still overlap as described herein in the event that these features overlap in at least one plane, such as a transverse plane including the transverse direction. Other suitable forms that staple cartridge (418) may take will be apparent to those of ordinary skill in the art in view of the teachings herein.

With respect to FIGS. 11-13, anvil (424) of the present example has a plurality of staple forming pockets (446). Each staple forming pocket (446) is positioned to lie over a corresponding staple pocket (442) of staple cartridge (418) when anvil (424) is in a closed position. Staple forming pockets (446) are configured to deform each leg (448) of staples (444) when staples (444) are driven through tissue and into anvil (424). In particular, staple forming pockets (446) are configured to bend legs (448) of staples (444) to secure the formed staples (444) in the tissue. Other suitable forms that anvil (424) may take will be apparent to those of ordinary skill in the art in view of the teachings herein.

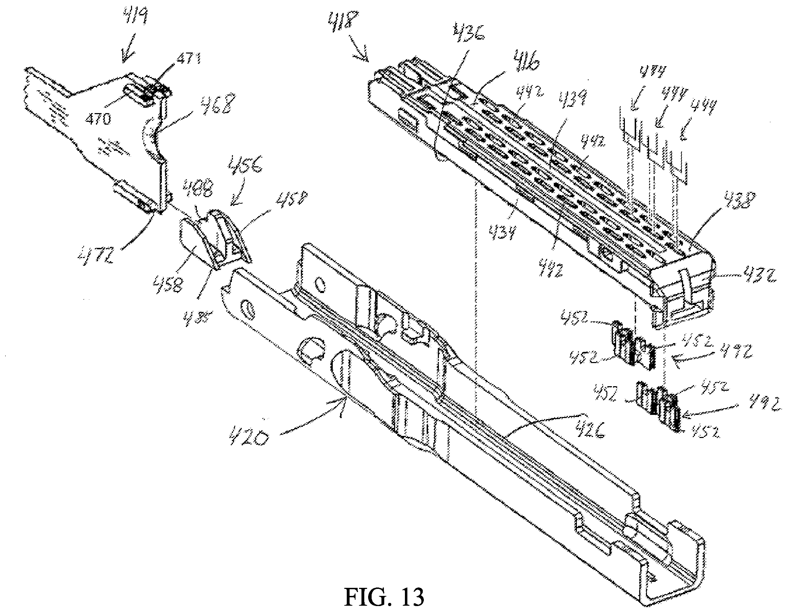

As best seen in FIG. 13, staple cartridge (418) includes staple drivers (452) positioned in staple pockets (442), underneath a corresponding staple (444), and above tray (436) *(see* FIG. 19). As will be described in greater detail below, staple drivers (452) are operable to translate upwardly in staple pockets (442) to thereby drive staples (444) upwardly through staple pockets (442) and into engagement with anvil (424). Staple drivers (452) are driven upwardly by a wedge sled (456), which is captured between cartridge body (434) and tray (436) (*see* FIG. 19), and which translates longitudinally through cartridge body (434) along a pair of cam slots (457). Wedge sled (456) includes a cam ramp (258) having a leading cam surface (460), an intermediate cam surface (462), and a trailing cam surface (464). By way of example only, leading cam surface (460) may be angled at approximately 45° relative to a horizontal plane; and intermediate cam surface (462) may be angled at approximately 22° relative to a horizontal plane. Alternatively, any other suitable angles may be used. Cam ramps (458) are generally configured to engage staple drivers (452) and thereby drive staple drivers (452) upwardly as wedge sled (456) translates longitudinally through staple cartridge (418) from a proximal sled position to a distal sled position. For instance, when wedge sled (456) is in the proximal sled position, staple drivers (452) are in downward positions and staples (444) are located in staple pockets (442). As wedge sled (456) is driven to the distal sled position by translating knife member (419), wedge sled (456) drives staple drivers (452) upwardly, thereby driving staples (444) out of staple pockets (442) and into staple forming pockets (446). Thus, staple drivers (452) translate along respective vertical planes as wedge sled (456) translates along a horizontal plane.

In the present example, knife member (419) is configured to translate through end effector (412). As best seen in FIG. 13, knife member (419) is secured to a distal end of firing beam (82), which extends through a portion of shaft assembly (30). Knife member (80) is positioned in channels (439, 441) of staple cartridge (418) and anvil (424), respectively. Knife member (419) includes a distally presented cutting edge (468) that is configured to sever tissue that is compressed between anvil (424) and deck (438) of staple cartridge (418) as knife member (419) translates distally through end effector (412). As noted above, knife member (419) also drives wedge sled (456) distally as knife member (419) translates distally through end effector (412), thereby driving staples (444) through tissue and against anvil (424) into formation.

### 1. Exemplary Triple Driver Assembly of Staple Cartridge

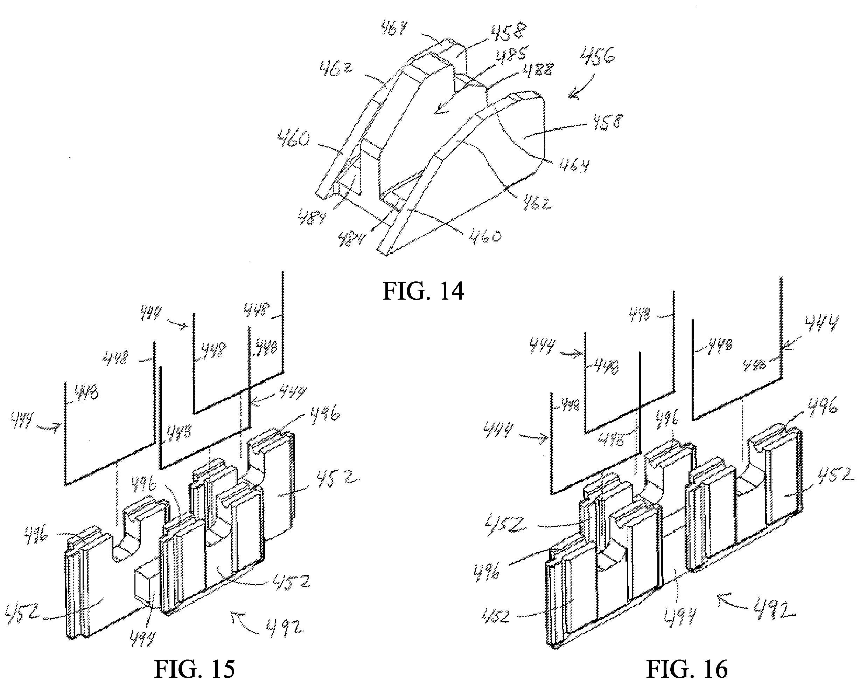

FIGS. 13-16 show wedge sled (456) and staple drivers (452), which are configured to direct staples (444) upwardly toward anvil (424) for forming staples (444) as described herein. Wedge sled (456) includes spacers (484) projecting from left and right sides of a central portion (485) thereof to a pair of left and right cam ramps (458). Spacers (484) are configured to center wedge sled (456) in a track slot (486) (*see* FIG. 19) extending through staple cartridge (418) along centerline (440). Each cam ramp (458) projects upwardly from each respective spacer (484) to align each cam ramp (458) centrally within parallel linear cam slots (457) as wedge sled (456) slides from the proximal sled position to the distal sled position. A rear end portion (488) receives translating knife member (419), which is configured to translate toward distal tip (432), for directing the wedge sled (456) distally toward the distal position.

A driver assembly (492) includes three staple drivers (452) connected by a driver cam (494) extending therebetween. As such, driver assembly (492) may also be referred to as triple driver assembly (492) Two of the three staple drivers (452) generally include a distally positioned staple driver (452) and a proximally positioned staple driver (452) on a common lateral side of driver cam (494) such that these staple drivers (452) are generally longitudinally aligned. These staple drivers (452) may also be referred to below more specifically as distal and proximal staple drivers (452). In addition, the third staple driver (452) may be referred to as intermediate staple driver (452) and overlaps between distal and proximal drivers (452) on an opposing side of driver cam (494).

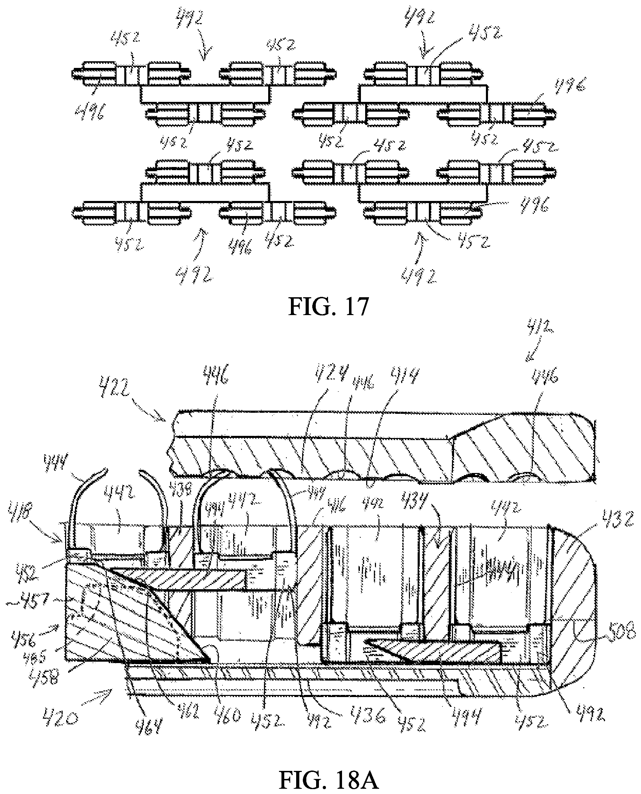

Each of the distal, intermediate, and proximal staple drivers (452) are in parallel with each other. In addition, each triple driver assembly (452) is configured to similarly overlap with another proximally positioned triple driver assembly (492) and another distally positioned triple driver assembly (492), as seen in FIG. 17. In other words, triple driver assemblies (492) are arranged in an alternative fashion in each row, such that one triple driver assembly (492) provides a single staple driver (452) on a first side of the row and two staple drivers (452) on a second side of the row; then the next triple driver assembly (492) provides a pair of staple drivers (452) on the first side of the row and a single staple driver (452) on the second side of the row; and so on. Triple driver assemblies (452) are thus aligned in alternating, asymmetric orientations in each row.

Each staple driver (452) further includes a longitudinal groove (496) that is configured to cradle the crown of a corresponding one of staples (444). It will be appreciated that each staple driver (452) may be secured to driver cam (494) relative to the other staple drivers (452) for triple driver assembly (492) to accommodate linear or arcuate portions of a variety of end effectors. As such, one of ordinary skill will appreciate the unique configurations of staple drivers (452) for sliding vertically through the plurality of staple pockets (442) aligned with staple forming pockets (446) (*see* FIG. 12) based on the descriptions herein. It will be further appreciated that the term "assembly" as used herein is not intended to be limited to discrete assembled components. Rather the term "assembly" includes components that may be formed separately and assembled and components that may be formed integrally as a single part. Thus, the term "assembly" is not intended to limit the invention described herein.

As shown in FIGS. 18A-19, cartridge body (434) defines elongated cam slots (457) that receive both cam ramp (458) of wedge sled (456) and driver cams (494) of each triple driver assembly (492) for engagement therebetween. Cam slots (457) extend through cartridge body (434) on opposing sides of centerline (440) such that wedge sled (456) straddles centerline (440) through central portion (485) and triple driver assemblies (492) are on each side of centerline (440). However, cam ramps (458) and driver cams (494) lie centrally along respective cam slots (457) such that each of the leading, intermediate, and trailing cam surfaces (460, 462, 464) successively engage driver cams (494) to direct each staple (444) upwardly toward anvil (460) for formation.

While various arrangements of staple drivers (452) are contemplated herein, triple driver assembly (492) of the present example has proximal, intermediate, and distal staple drivers (452) positioned such that distal staple driver (452) is cantilevered distally from driver cam (492). More particularly, this cantilever arrangement of distal driver cam (492) increases the distal most position of staple (444) cradled therein to effectively elongate triple driver assembly (492) to provide additional space for wedge sled (456). As such, staples pockets (442) and staples (444) may be positioned more closely to distal tip (432).

In use, FIG. 17 shows a top view of two pairs of exemplary triple driver assemblies (492) overlapped in the transverse direction and on opposing sides of centerline (430) to represent approximate positions within the plurality of staple pockets (442) as shown in FIG. 18A. In order to drive triple driver assemblies (492) upwardly toward anvil (424) for forming staples (444), translating knife member (419) forces wedge sled (456) distally to engage driver cam (494). Leading cam surface (460) of cam ramp (458) slides under driver cam (494) and lifts driver cam (494) vertically upwardly along the relatively steep angle of leading cam surface (460). Given the relatively steep angle of leading cam surface (460), the vertical movement is relatively large in view of the relatively small distance that cam ramps (458) slid along through cam slots (457).

As knife member (419) drives wedge sled (456) further distally, intermediate cam surfaces (462) of cam ramps (458) then slide under driver cams (494) and lift driver cams (494) further vertically upwardly along the relatively gradual angle of intermediate cam surface (460). The relatively gradual angle of intermediate cam surface (462) lifts triple driver assemblies (492) a relatively small vertical distance in view of the relatively large distance that cam ramps (458) slide through cam slots (457). Thereby, wedge sled (456) is configured to complete the work to form staple (444) within tissue with less force by taking advantage of the known principle that increasing distance over which a force is applied allows equivalent work to be done with less force.

With the staples (444) formed on each side of centerline (440) as shown in FIG. 18B, translating knife member (419) cuts tissue while simultaneously directing wedge sled (456) to continue to slide distally along centerline (440) such that trailing cam surfaces (464) provide any further upward force necessary to inhibit staples (444) and/or staple drivers (452) from recoiling vertically downwardly. In some versions, trailing cam surface (464) is generally horizontal. Wedge sled (456) continues to slide distally toward the distal position along track slot (486) and cam slots (457) to further drive upward movement of triple staple driver assemblies (492) throughout the remaining length of end effector (412).

### 2. Exemplary Method of Tissue Resection

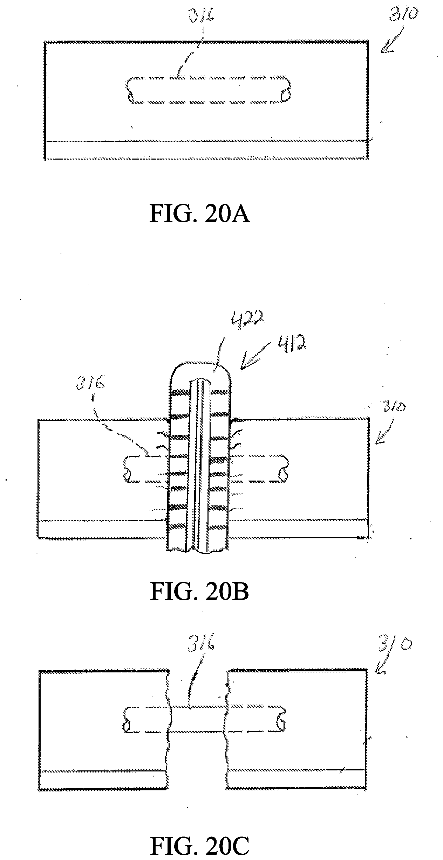

FIGS. 20A-20E show one example of using end effector (412) to resect tissue, such as a liver parenchyma tissue (310), and to ligate a vessel or duct (316) therein. As noted above, vessel or duct (316) may comprise a hepatic vein or a hepatic artery. It should also be understood that the method may further include the use of end effector (412) to ligate other vessels such as the portal vein and extrahepatic vessels, etc.

As shown in FIG. 20B, the operator positions end effector (412) such that tissue (310), including vessel or duct (316), is located between lower and upper jaws (420, 422). The operator then compresses tissue (310) between upper and lower crush surfaces (414, 416) of upper and lower jaws (420, 422), respectively, to deliver the predetermined crush pressure to tissue (310). By way of example only, jaws (420, 422) may be actuated in this manner by pivoting trigger (24) toward pistol grip (22). It should be understood that jaws (420, 422) need not necessarily be actuated to a fully closed configuration. In some instances, the operator may rely on tactile feedback through trigger (24) and pistol grip (22) to determine whether the operator has achieved a desired gap between jaws (420,422) to suitably crush tissue (310) without undesirably damaging vessel or duct (316). In addition or in the alternative, the operator may rely on visual feedback.

In any case, the crush pressure applied by jaws (420, 422) effectively severs tissue (310), and the operator then removes end effector (412) from tissue (310) to view whether or not any vessels or ducts are present. As shown in FIG. 20C, vessel or duct (316) remains intact and is left exposed, extending between severed portions of tissue (310).

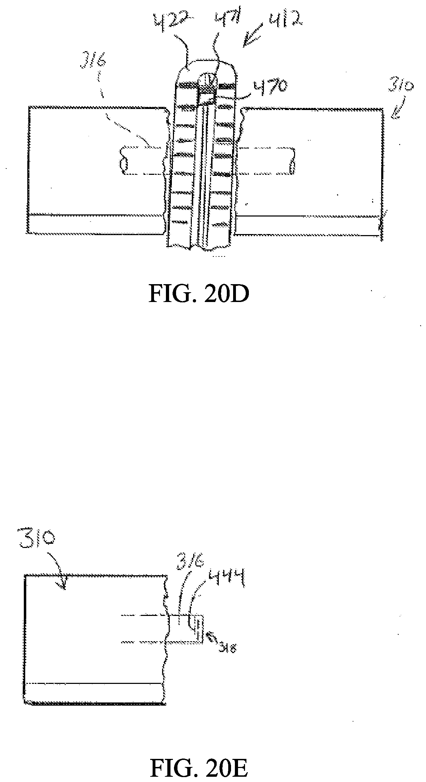

In some instances, the operator may leave vessel or duct (316) intact. However, in the present example, the operator ligates vessel or duct (316) to complete the resection of a severed portion of tissue (310), as shown in FIG. 20D. Ligation includes placement of at least some of overlapping staples (444) within vessel or duct (316) as discussed above in greater detail. It should therefore be understood that the same end effector (412) may be used to crush (and thereby sever) tissue (310) of the liver and also ligate a vessel or duct (316) in the tissue (310). In the present example, the vessel or duct (316) is stapled and severed substantially simultaneously by end effector (412), resulting in the configuration shown in FIG. 20E. As shown, the severed end (318) of the vessel or duct (316) is sealed by staples (444). Thereby, the operator completes resection of a right portion of tissue (310) and the corresponding portion of the vessel or duct (316).

As described above, the operator removes end effector (412) for viewing vessel (316) as shown in FIG. 20C. Alternatively, the operator may apply the predetermined crush pressure (or as determined based on tactile and/or visual feedback as noted above), as shown in FIG. 20B, and immediately thereafter sever and ligate any tissue remaining therein, such as vessel or duct (316). As such, it is not necessary to view such tissue, but the operator may find such viewing desirable in one or more liver resection procedures. It will be appreciated that the above described resection is merely illustrative and not limited to liver tissue. Alternatively, tissue resection with end effector (412) may be performed on other tissues within the patient as desired by the user.

### III. Exemplary Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### IV. Miscellaneous

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

Versions of the devices described above may have application in conventional medical treatments and procedures conducted by a medical professional, as well as application in robotic-assisted medical treatments and procedures. By way of example only, various teachings herein may be readily incorporated into a robotic surgical system such as the DAVINCI™ system by Intuitive Surgical, Inc., of Sunnyvale, California. Similarly, those of ordinary skill in the art will recognize that various teachings herein may be readily combined with various teachings of any of the following: U.S. Pat. No. 5,792,135, entitled "Articulated Surgical Instrument For Performing Minimally Invasive Surgery With Enhanced Dexterity and Sensitivity," issued August 11, 1998; U.S. Pat. No. 5,817,084, entitled "Remote Center Positioning Device with Flexible Drive," issued October 6, 1998; U.S. Pat. No. 5,878,193, entitled "Automated Endoscope System for Optimal Positioning," issued March 2, 1999; U.S. Pat. No. 6,231,565, entitled "Robotic Arm DLUS for Performing Surgical Tasks," issued May 15, 2001; U.S. Pat. No. 6,783,524, entitled "Robotic Surgical Tool with Ultrasound Cauterizing and Cutting Instrument," issued August 31, 2004; U.S. Pat. No. 6,364,888, entitled "Alignment of Master and Slave in a Minimally Invasive Surgical Apparatus," issued April 2, 2002; U.S. Pat. No. 7,524,320, entitled "Mechanical Actuator Interface System for Robotic Surgical Tools," issued April 28, 2009; U.S. Pat. No. 7,691,098, entitled "Platform Link Wrist Mechanism," issued April 6, 2010; U.S. Pat. No. 7,806,891, entitled "Repositioning and Reorientation of Master/Slave Relationship in Minimally Invasive Telesurgery," issued October 5, 2010; U.S. Pub. No. 2013/0012957, entitled "Automated End Effector Component Reloading System for Use with a Robotic System, published January 10, 2013; U.S. Pub. No. 2012/0199630, entitled "Robotically-Controlled Surgical Instrument with Force-Feedback Capabilities," published August 9, 2012; U.S. Pub. No. 2012/0132450, entitled "Shiftable Drive Interface for Robotically-Controlled Surgical Tool," published May 31, 2012; U.S. Pub. No. 2012/0199633, entitled "Surgical Stapling Instruments with Cam-Driven Staple Deployment Arrangements," published August 9, 2012; U.S. Pub. No. 2012/0199631, entitled "Robotically-Controlled Motorized Surgical End Effector System with Rotary Actuated Closure Systems Having Variable Actuation Speeds," published August 9, 2012; U.S. Pub. No. 2012/0199632, entitled "Robotically-Controlled Surgical Instrument with Selectively Articulatable End Effector," published August 9, 2012; U.S. Pub. No. 2012/0203247, entitled "Robotically-Controlled Surgical End Effector System," published August 9, 2012; U.S. Pub. No. 2012/0211546, entitled "Drive Interface for Operably Coupling a Manipulatable Surgical Tool to a Robot," published August 23, 2012; U.S. Pub. No. 2012/0138660, entitled "Robotically-Controlled Cable-Based Surgical End Effectors," published June 7, 2012; and/or U.S. Pub. No. 2012/0205421, entitled "Robotically-Controlled Surgical End Effector System with Rotary Actuated Closure Systems," published August 16, 2012.

Versions of the devices described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by a operator immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometries, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A surgical instrument (10; 410) for treating a tissue of a patient, comprising:
(a) a shaft assembly (30);
(b) an end effector (40; 412) extending from the shaft assembly along a jaw centerline (440), the end effector comprising:
(i) a first jaw (422) having an anvil (60; 424) configured to form a plurality of staples (77, 444) pressed against the anvil, and
(ii) a second jaw (50; 420), wherein the first and second jaws are configured to transition between an open configuration and a closed configuration; and
(c) a staple cartridge (70; 418) received within the second jaw, the staple cartridge comprising:
(i) a deck (73; 438) facing the anvil, wherein the deck defines a plurality of staple openings (74; 442), the plurality of staple openings comprising a first row of staple openings and a second row of staple openings, the first and second rows of staple openings defining a first row centerline therebetween,
(ii) a plurality of staples positioned respectively within the plurality of staple openings,
(iii) a first driver assembly (75; 492) positioned on the first row centerline, wherein the first driver assembly is asymmetric about the first row centerline and is configured to drive a first portion of the plurality of staples, and
(iv) a second driver assembly (75; 492) positioned on the first row centerline, wherein the second driver assembly is asymmetric about the first row centerline and is configured to drive a second portion of the plurality of staples;
wherein the first and second driver assemblies alternate such that the first and second driver assemblies overlap in a direction transverse to the first row centerline for forming offset and overlapping staple rows in tissue.

2. The surgical instrument of claim 1, wherein the staple cartridge further comprises a wedge sled configured to slide proximate to the deck from a proximal sled position to a distal sled position such that the wedge sled is configured to progressively engage the first and second driver assemblies sliding toward the distal sled position and progressively force the first and second portions of the plurality of staples toward the anvil for formation in the tissue.

3. The surgical instrument of claim 2, wherein the wedge sled has a first cam ramp, wherein the first cam ramp is configured to engage the first and second driver assemblies.

4. The surgical instrument of claim 3, wherein the plurality of staple openings include a third row of staple openings and a fourth row of staple openings, the third and fourth row of staple openings defining a second row centerline therebetween, wherein the first and second row centerlines are offset from each other and extending along opposing sides of the jaw centerline, wherein the staple cartridge further comprises:
(i) a third driver assembly positioned on the second row centerline, wherein the third driver assembly is asymmetric about the second row centerline and is configured to drive a third portion of the plurality of staples, and
(ii) a fourth driver assembly positioned on the row centerline and being asymmetric about the second row centerline and configured to drive a fourth portion of the plurality of staples;
wherein the wedge sled has a second cam ramp, wherein the second cam ramp is configured to progressively engage the third and fourth driver assemblies.

5. The surgical instrument of any one of claims 2 to 4, wherein the wedge sled has a distal nose, wherein the second jaw has a blocker wall distally positioned therein along the centerline, wherein the blocker wall is configured to receive the wedge sled thereagainst and inhibit movement of the wedge sled distally beyond the distal sled position, wherein the blocker wall defines a clearance hole configured to receive the distal nose of the wedge sled in the distal sled position.

6. The surgical instrument of claim 5 when dependent on claim 3 or claim 4, wherein the second driver assembly is a distal-most driver assembly, wherein a majority of the first cam ramp in the distal sled position is below the distal-most driver assembly.

7. The surgical instrument of any one of claims 1 to 6, wherein the first row centerline is offset from the jaw centerline.

8. The surgical instrument of claim 7, wherein the first row centerline is parallel with the jaw centerline.

9. The surgical instrument of any one of claims 1 to 8, wherein the first driver assembly has a first distal driver, a first intermediate driver, and a first proximal driver;
wherein the first distal driver, the first intermediate driver, and the first proximal drivers are operatively connected such that the first distal driver and the first proximal driver are longitudinally aligned and the first intermediate driver is transversely offset from each of the first distal driver and the first proximal driver;
wherein the first distal driver, the first intermediate driver, and the first proximal driver respectively receive the first portion of the plurality of staples.

10. The surgical instrument of claim 9, wherein the second driver assembly has a second distal driver, a second intermediate driver, and a second proximal driver;
wherein the second distal driver, the second intermediate driver, and the second proximal drivers are operatively connected such that the second distal driver and the second proximal driver are longitudinally aligned and the second intermediate driver is transversely offset from each of the second distal driver and the second proximal driver;
wherein the second distal driver, the second intermediate driver, and the second proximal driver respectively receive the second portion of the plurality of staples.

11. The surgical instrument of claim 10, wherein the first and second driver assemblies alternate such that first and second intermediate drivers of the respective first and second driver assemblies are positioned on opposing sides of the first row centerline.

12. The surgical instrument of claim 11, wherein the first distal driver of the first driver assembly overlaps in the direction transverse to the first row centerline with the second proximal driver of the second driver assembly.

13. The surgical instrument of any one of claims 9 to 12, wherein the first distal driver, the first intermediate driver, and the first proximal driver are connected by a first driver cam extending therebetween.

14. The surgical instrument of claim 13, wherein the staple cartridge further comprises a wedge sled configured to slide proximate to the deck from a proximal sled position to a distal sled position such that the wedge sled is configured to engage the first driver cam thereby forcing the first distal driver, the first intermediate driver, and the first proximal driver toward the anvil for forming the first portion of the plurality of staples against the anvil.

15. The surgical instrument of any one of claims 1 to 14, wherein the first jaw has a first elongated channel extending therethrough and a first plurality of indicia, wherein the second jaw has a second elongated channel extending therethrough and a second plurality of indicia, the end effector further comprising a knife member received within the first and second channels, wherein the knife member is configured to slide distally along the first and second channels and engage a wedge sled for forming staples, wherein the knife member comprises an first indicator in the first channel and a second indicator in the first channel, and wherein the first and second indicators in conjunction with the first and second plurality of indicia are respectively configured to indicate a staple usage to an operator.

## Patentansprüche

1. Chirurgisches Instrument (10; 410) zur Behandlung eines Gewebes eines Patienten, umfassend:
(a) eine Schaftanordnung (30),
(b) einen Endeffektor (40; 412), der sich von der Schaftanordnung entlang einer Backenmittellinie (440) erstreckt, wobei der Endeffektor Folgendes umfasst:
(i) eine erste Backe (422) mit einem Amboss (60; 424), der dazu ausgestaltet ist, eine Vielzahl von gegen den Amboss gedrückten Klammern (77, 444) zu bilden, und
(ii) eine zweite Backe (50; 420), wobei die erste und die zweite Backe dazu ausgestaltet sind, zwischen einer offenen Konfiguration und einer geschlossenen Konfiguration überzugehen, und
(c) ein in der zweiten Backe aufgenommenes Klammermagazin (70; 418), wobei das Klammermagazin Folgendes umfasst:
(i) eine dem Amboss zugewandte Plattform (73; 438), wobei die Plattform eine Vielzahl von Klammeröffnungen (74; 442) definiert, wobei die Vielzahl von Klammeröffnungen eine erste Reihe von Klammeröffnungen und eine zweite Reihe von Klammeröffnungen umfasst, wobei die erste und die zweite Reihe von Klammeröffnungen dazwischen eine erste Reihenmittellinie definieren,
(ii) eine Vielzahl von jeweils in der Vielzahl von Klammeröffnungen positionierten Klammern,
(iii) eine erste Treiberanordnung (75; 492), die auf der ersten Reihenmittellinie positioniert ist, wobei die erste Treiberanordnung asymmetrisch zu der ersten Reihenmittellinie ist und dazu ausgestaltet ist, einen ersten Teil der Vielzahl von Klammern zu treiben, und
(iv) eine zweite Treiberanordnung (75; 492), die auf der ersten Reihenmittellinie positioniert ist, wobei die zweite Treiberanordnung asymmetrisch zu der ersten Reihenmittellinie ist und dazu ausgestaltet ist, einen zweiten Teil der Vielzahl von Klammern zu treiben,
wobei die erste und die zweite Treiberanordnung abwechseln, so dass die erste und die zweite Treiberanordnung in einer quer zu der ersten Reihenmittellinie verlaufenden Richtung überlappen, um versetzte und überlappende Klammerreihen in Gewebe zu bilden.

2. Chirurgisches Instrument nach Anspruch 1, wobei das Klammermagazin ferner einen Keilschlitten umfasst, der dazu ausgestaltet ist, proximal zu der Plattform aus einer proximalen Schlittenposition in eine distale Schlittenposition zu gleiten, so dass der Keilschlitten dazu ausgestaltet ist, die erste und die zweite Treiberanordnung fortschreitend in Eingriff zu nehmen, während er zu der distalen Schlittenposition hin gleitet, und den ersten und den zweiten Teil der Vielzahl von Klammern zum Bilden im Gewebe fortschreitend zu dem Amboss hin zu zwingen.

3. Chirurgisches Instrument nach Anspruch 2, wobei der Keilschlitten eine erste Nockenramp hat, wobei die erste Nockenrampe dazu ausgestaltet ist, die erste und die zweite Treiberanordnung in Eingriff zu nehmen.

4. Chirurgisches Instrument nach Anspruch 3, wobei die Vielzahl von Klammeröffnungen eine dritte Reihe von Klammeröffnungen und eine vierte Reihe von Klammeröffnungen aufweist, wobei die dritte und die vierte Reihe von Klammeröffnungen eine zweite Reihenmittellinie dazwischen definieren, wobei die erste und die zweite Reihenmittellinie voneinander versetzt sind und sich entlang gegenüberliegenden Seiten der Backenmittellinie erstrecken, wobei das Klammermagazin ferner Folgendes umfasst:
(i) eine auf der zweiten Reihenmittellinie positionierte dritte Treiberanordnung, wobei die dritte Treiberanordnung asymmetrisch zu der zweiten Reihenmittellinie ist und dazu ausgestaltet ist, einen dritten Teil der Vielzahl von Klammern zu treiben, und
(ii) eine vierte Treiberanordnung, die auf der Reihenmittellinie positioniert und asymmetrisch zu der zweiten Reihenmittellinie ist und dazu ausgestaltet ist, einen vierten Teil der Vielzahl von Klammern zu treiben,
wobei der Keilschlitten eine zweite Nockenrampe hat, wobei die zweite Nockenrampe dazu ausgestaltet ist, die dritte und die vierte Treiberanordnung fortschreitend in Eingriff zu nehmen.

5. Chirurgisches Instrument nach einem der Ansprüche 2 bis 4, wobei der Keilschlitten eine distale Nase hat, wobei die zweite Backe eine Blockierwand hat, die distal darin entlang der Mittellinie positioniert ist, wobei die Blockierwand dazu ausgestaltet ist, den Keilschlitten daran aufzunehmen und eine distal über die distale Schlittenposition hinausgehende Bewegung des Keilschlittens zu hemmen, wobei die Blockierwand ein Freiraumloch definiert, das dazu ausgestaltet ist, die distale Nase des Keilschlittens in der distalen Schlittenposition aufzunehmen.

6. Chirurgisches Instrument nach Anspruch 5, wenn er von Anspruch 3 oder Anspruch 4 abhängig ist, wobei die zweite Treiberanordnung eine distalste Treiberanordnung ist, wobei ein Großteil der ersten Nockenrampe in der distalen Schlittenposition unter der distalsten Treiberanordnung liegt.

7. Chirurgisches Instrument nach einem der Ansprüche 1 bis 6, wobei die erste Reihenmittellinie von der Backenmittellinie versetzt ist.

8. Chirurgisches Instrument nach Anspruch 7, wobei die erste Reihenmittellinie parallel zu der Backenmittellinie verläuft.

9. Chirurgisches Instrument nach einem der Ansprüche 1 bis 8, wobei die erste Treiberanordnung einen ersten distalen Treiber, einen ersten Zwischentreiber und einen ersten proximalen Treiber hat,
wobei der erste distale Treiber, der erste Zwischentreiber und der erste proximale Treiber wirkverbunden sind, so dass der erste distale Treiber und der erste proximale Treiber in Längsrichtung ausgerichtet sind und der erste Zwischentreiber von jeweils dem ersten distalen Treiber und dem ersten proximalen Treiber quer versetzt ist,
wobei der erste distale Treiber, der erste Zwischentreiber und der erste proximale Treiber jeweils den ersten Teil der Vielzahl von Klammern aufnehmen.

10. Chirurgisches Instrument nach Anspruch 9, wobei die zweite Treiberanordnung einen zweiten distalen Treiber, einen zweiten Zwischentreiber und einen zweiten proximalen Treiber hat,
wobei der zweite distale Treiber, der zweite Zwischentreiber und der zweite proximale Treiber wirkverbunden sind, so dass der zweite distale Treiber und der zweite proximale Treiber in Längsrichtung ausgerichtet sind und der zweite Zwischentreiber von jeweils dem zweiten distalen Treiber und dem zweiten proximalen Treiber quer versetzt ist,
wobei der zweite distale Treiber, der zweite Zwischentreiber und der zweite proximale Treiber jeweils den zweiten Teil der Vielzahl von Klammern aufnehmen.

11. Chirurgisches Instrument nach Anspruch 10, wobei die erste und die zweite Treiberanordnung abwechseln, so dass der erste und zweite Zwischentreiber der jeweiligen ersten und zweiten Treiberanordnung auf gegenüberliegenden Seiten der ersten Reihenmittellinie positioniert sind.

12. Chirurgisches Instrument nach Anspruch 11, wobei der erste distale Treiber der ersten Treiberanordnung in der quer zu der ersten Reihenmittellinie verlaufenden Richtung den zweiten proximalen Treiber der zweiten Treiberanordnung überlappt.

13. Chirurgisches Instrument nach einem der Ansprüche 9 bis 12, wobei der erste distale Treiber, der erste Zwischentreiber und der erste proximale Treiber über einen sich dazwischen erstreckenden ersten Treibernocken verbunden sind.

14. Chirurgisches Instrument nach Anspruch 13, wobei das Klammermagazin ferner einen Keilschlitten umfasst, der dazu ausgestaltet ist, proximal zu der Plattform aus einer proximalen Schlittenposition in eine distale Schlittenposition zu gleiten, so dass der Keilschlitten dazu ausgestaltet ist, den ersten Treibernocken in Eingriff zu nehmen, wodurch der erste distale Treiber, der erste Zwischentreiber und der erste proximale Treiber zum Amboss hin gezwungen werden, um den ersten Teil der Vielzahl von Klammern gegen den Amboss zu bilden.

15. Chirurgisches Instrument nach einem der Ansprüche 1 bis 14, wobei die erste Backe einen sich dort hindurcherstreckenden ersten länglichen Kanal und eine erste Vielzahl von Markierungen hat, wobei die zweite Backe einen sich dort hindurcherstreckenden zweiten länglichen Kanal und eine zweite Vielzahl von Markierungen hat, wobei der Endeffektor ferner ein in dem ersten und dem zweiten Kanal aufgenommenes Messerglied umfasst, wobei das Messerglied dazu ausgestaltet ist, distal entlang dem ersten und dem zweiten Kanal zu gleiten und zum Bilden von Klammern einen Keilschlitten in Eingriff zu nehmen, wobei das Messerglied eine erste Anzeige in dem ersten Kanal und eine zweite Anzeige in dem ersten Kanal umfasst und wobei die erste und die zweite Anzeige in Verbindung mit der ersten und der zweiten Vielzahl von Markierungen jeweils dazu ausgestaltet sind, einer Bedienperson gegenüber eine Klammerbenutzung anzugeben.

## Revendications

1. Instrument chirurgical (10 ; 410) pour traiter un tissu d'un patient, comprenant :
(a) un ensemble d'arbre (30) ;
(b) un effecteur terminal (40 ; 412) s'étendant depuis l'ensemble d'arbre le long d'un axe central de mâchoire (440), l'effecteur terminal comprenant :
(i) une première mâchoire (422) ayant une enclume (60 ; 424) configurée pour former une pluralité d'agrafes (77, 444) pressées contre l'enclume, et
(ii) une deuxième mâchoire (50 ; 420), la première et la deuxième mâchoire étant configurées pour commuter entre une configuration ouverte et une configuration fermée ; et
(c) une cartouche d'agrafes (70 ; 418) reçue à l'intérieur de la deuxième mâchoire, la cartouche d'agrafes comprenant :
(i) un plateau (73 ; 438) faisant face à l'enclume, le plateau définissant une pluralité d'ouvertures d'agrafes (74 ; 442), la pluralité d'ouvertures d'agrafes comprenant une première rangée d'ouvertures d'agrafes et une deuxième rangée d'ouvertures d'agrafes, la première et la deuxième rangée d'ouvertures d'agrafes définissant un premier axe central de rangées entre elles,
(ii) une pluralité d'agrafes positionnées respectivement à l'intérieur de la pluralité d'ouvertures d'agrafes,
(iii) un premier ensemble d'enfoncement (75 ; 492) positionné sur le premier axe central de rangées, le premier ensemble d'enfoncement étant asymétrique autour du premier axe central de rangées et étant configuré pour enfoncer une première partie de la pluralité d'agrafes, et
(iv) un deuxième ensemble d'enfoncement (75 ; 492) positionné sur le premier axe central de rangées, le deuxième ensemble d'enfoncement étant asymétrique autour du premier axe central de rangées et étant configuré pour enfoncer une deuxième partie de la pluralité d'agrafes ;
dans lequel le premier et le deuxième ensemble d'enfoncement alternent de telle sorte que le premier et le deuxième ensemble d'enfoncement se chevauchent dans une direction transversale au premier axe central de rangées pour former des rangées d'agrafes décalées et se chevauchant dans les tissus.

2. Instrument chirurgical selon la revendication 1, dans lequel la cartouche d'agrafes comprend en outre un chariot cunéiforme configuré pour coulisser à proximité du plateau depuis une position de chariot proximale jusqu'à une position de chariot distale de telle sorte que le chariot cunéiforme soit configuré pour s'engager progressivement avec le premier et le deuxième ensemble d'enfoncement coulissant vers la position de chariot distale et pour forcer progressivement la première et la deuxième partie de la pluralité d'agrafes vers l'enclume en vue de la formation dans les tissus.

3. Instrument chirurgical selon la revendication 2, dans lequel le chariot cunéiforme présente une première rampe de came, la première rampe de came étant configurée pour s'engager avec le premier et le deuxième ensemble d'enfoncement.

4. Instrument chirurgical selon la revendication 3, dans lequel la pluralité d'ouvertures d'agrafes comporte une troisième rangée d'ouvertures d'agrafes et une quatrième rangée d'ouvertures d'agrafes, la troisième et la quatrième rangée d'ouvertures d'agrafes définissant un deuxième axe central de rangées entre elles, le premier et le deuxième axe central de rangées étant décalés l'un de l'autre et s'étendant le long de côtés opposés de l'axe central de mâchoire, la cartouche d'agrafes comprenant en outre :
(i) un troisième ensemble d'enfoncement positionné sur le deuxième axe central de rangées, le troisième ensemble d'enfoncement étant asymétrique autour du deuxième axe central de rangées et étant configuré pour enfoncer une troisième partie de la pluralité d'agrafes, et
(ii) un quatrième ensemble d'enfoncement positionné sur l'axe central de rangées et étant asymétrique autour du deuxième axe central de rangées et étant configuré pour enfoncer une quatrième partie de la pluralité d'agrafes ;
le chariot cunéiforme ayant une deuxième rampe de came, la deuxième rampe de came étant configurée pour s'engager progressivement avec le troisième et le quatrième ensemble d'enfoncement.

5. Instrument chirurgical selon l'une quelconque des revendications 2 à 4, dans lequel le chariot cunéiforme présente un nez distal, la deuxième mâchoire ayant une paroi de blocage positionnée distalement dans celle-ci le long de l'axe central, la paroi de blocage étant configurée pour recevoir le chariot cunéiforme contre celle-ci et pour empêcher le mouvement du chariot cunéiforme dans la direction distale au-delà de la position de chariot distale, la paroi de blocage définissant un trou de dégagement configuré pour recevoir le nez distal du chariot cunéiforme dans la position de chariot distale.

6. Instrument chirurgical selon la revendication 5 lorsqu'elle dépend de la revendication 3 ou de la revendication 4, dans lequel le deuxième ensemble d'enfoncement est un ensemble d'enfoncement le plus distal, une majorité de la première rampe de came dans la position de chariot distale étant en dessous de l'ensemble d'enfoncement le plus distal.

7. Instrument chirurgical selon l'une quelconque des revendications 1 à 6, dans lequel le premier axe central de rangées est décalé par rapport à l'axe central de mâchoire.

8. Instrument chirurgical selon la revendication 7, dans lequel le premier axe central de rangées est parallèles à l'axe central de mâchoire.

9. Instrument chirurgical selon l'une quelconque des revendications 1 à 8, dans lequel le premier ensemble d'enfoncement présente un premier dispositif d'enfoncement distal, un premier dispositif d'enfoncement intermédiaire et un premier dispositif d'enfoncement proximal ;
le premier dispositif d'enfoncement distal, le premier dispositif d'enfoncement intermédiaire et le premier dispositif d'enfoncement proximal étant connectés fonctionnellement de telle sorte que le premier dispositif d'enfoncement distal et le premier dispositif d'enfoncement proximal soient alignés longitudinalement et que le premier dispositif d'enfoncement intermédiaire soit décalé transversalement par rapport à chacun parmi le premier dispositif d'enfoncement distal et le premier dispositif d'enfoncement proximal ;
le premier dispositif d'enfoncement distal, le premier dispositif d'enfoncement intermédiaire et le premier dispositif d'enfoncement proximal recevant respectivement la première partie de la pluralité d'agrafes.

10. Instrument chirurgical selon la revendication 9, dans lequel le deuxième ensemble d'enfoncement présente un deuxième dispositif d'enfoncement distal, un deuxième dispositif d'enfoncement intermédiaire et un deuxième dispositif d'enfoncement proximal ;
le deuxième dispositif d'enfoncement distal, le deuxième dispositif d'enfoncement intermédiaire et le deuxième dispositif d'enfoncement proximal étant connectés fonctionnellement de telle sorte que le deuxième dispositif d'enfoncement distal et le deuxième dispositif d'enfoncement proximal soient alignés longitudinalement et que le deuxième dispositif d'enfoncement intermédiaire soit décalé transversalement par rapport à chacun parmi le deuxième dispositif d'enfoncement distal et le deuxième dispositif d'enfoncement proximal ; le deuxième dispositif d'enfoncement distal, le deuxième dispositif d'enfoncement intermédiaire et le deuxième dispositif d'enfoncement proximal recevant respectivement la deuxième partie de la pluralité d'agrafes.

11. Instrument chirurgical selon la revendication 10, dans lequel le premier et le deuxième ensemble d'enfoncement alternent de telle sorte que le premier et le deuxième dispositif d'enfoncement intermédiaire parmi le premier et le deuxième ensemble d'enfoncement respectif soient positionnés sur des côtés opposés du premier axe central de rangées.

12. Instrument chirurgical selon la revendication 11, dans lequel le premier dispositif d'enfoncement distal du premier ensemble d'enfoncement chevauche le deuxième dispositif d'enfoncement proximal du deuxième ensemble d'enfoncement dans la direction transversale au premier axe central de rangées.

13. Instrument chirurgical selon l'une quelconque des revendications 9 à 12, dans lequel le premier dispositif d'enfoncement distal, le premier dispositif d'enfoncement intermédiaire et le premier dispositif d'enfoncement proximal sont connectés par une première came de dispositifs d'enfoncement s'étendant entre eux.

14. Instrument chirurgical selon la revendication 13, dans lequel la cartouche d'agrafes comprend en outre un chariot cunéiforme configuré pour coulisser à proximité du plateau depuis une position de chariot proximale jusqu'à une position de chariot distale de telle sorte que le chariot cunéiforme soit configuré pour s'engager avec la première came de dispositifs d'enfoncement pour ainsi forcer le premier dispositif d'enfoncement distal, le premier dispositif d'enfoncement intermédiaire et le premier dispositif d'enfoncement proximal vers l'enclume pour former la première partie de la pluralité d'agrafes contre l'enclume.

15. Instrument chirurgical selon l'une quelconque des revendications 1 à 14, dans lequel la première mâchoire présente un premier canal allongé s'étendant à travers elle et une première pluralité d'inscriptions, la deuxième mâchoire présente un deuxième canal allongé s'étendant à travers elle et une deuxième pluralité d'inscriptions, l'effecteur terminal comprenant en outre un organe de couteau reçu à l'intérieur du premier et du deuxième canal, l'organe de couteau étant configuré pour coulisser dans la direction distale le long du premier et du deuxième canal et pour s'engager avec un chariot cunéiforme pour former des agrafes, l'organe de couteau comprenant un premier indicateur dans le premier canal et un deuxième indicateur dans le deuxième canal, et le premier et le deuxième indicateur, conjointement avec la première et la deuxième pluralité d'inscriptions, étant configurés respectivement pour indiquer une utilisation d'agrafes à un opérateur.
